(19) **Europäisches Patentamt**
**European Patent Office**
**Office européen des brevets**

(11) **EP 1 154 744 B1**

(12) **EUROPEAN PATENT SPECIFICATION**

(45) Date of publication and mention
of the grant of the patent:
**16.01.2008 Bulletin 2008/03**

(51) Int Cl.:
***A61F 13/15*** (2006.01)

(21) Application number: **99963050.2**

(86) International application number:
**PCT/US1999/029130**

(22) Date of filing: **08.12.1999**

(87) International publication number:
**WO 2000/033779 (15.06.2000 Gazette 2000/24)**

(54) **MULTI-LAYER LINERS FOR PERSONAL CARE PRODUCTS**

MEHRSCHICHTIGE EINLAGE FÜR KÖRPERPFLEGEPRODUKTE

REVETEMENTS MULTICOUCHE POUR ARTICLES DE SOINS PERSONNELS

(84) Designated Contracting States:
**DE ES FR GB IT**

(30) Priority: **09.12.1998 US 209177**

(43) Date of publication of application:
**21.11.2001 Bulletin 2001/47**

(73) Proprietor: **KIMBERLY-CLARK WORLDWIDE, INC.**
**Neenah, Wisconsin 54956 (US)**

(72) Inventors:
• **VARONA, Eugenio, Go**
**Marietta, GA 30062 (US)**

• **KEPNER, Eric, Scott**
**Fletcher, NC 28732 (US)**
• **SMITH, Jr., Roland, Columbus**
**Gainesville, GA 30507 (US)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**EP-A- 0 672 774        WO-A-98/22066**
**US-A- 5 192 606        US-A- 5 820 973**
**US-A- 5 843 063**

## Description

This application is being filed the same day as a co-assigned case having attorney docket number 13919 entitled "Creped Materials".

## FIELD OF THE INVENTION

The present invention relates to a liner mainly for personal care products like diapers, training pants, swim wear, absorbent underpants, adult incontinence products, bandages and feminine hygiene products. This material may also be used in bandages and wound dressings, nursing pads and veterinary applications.

## BACKGROUND OF THE INVENTION

Personal care articles, such as diapers, typically are composed of multiple components including the liner, absorbent core, and baffle (also called a backsheet) The liner in conjunction with an absorbent layer(s) often must deliver softness and comfort, visual distinctiveness, absorbency, cleanliness, and dryness. The extent to which these traits are met is dependent on the structure and surface chemistry of the topsheet and the absorbent core.

The liner is sometimes referred to as a bodyside liner or topsheet and is usually adjacent a surge material. In the thickness direction of the article, the liner material is the layer against the wearer's skin and so the first layer in contact with liquid or other exudate from the wearer. The liner further serves to isolate the wearer's skin from the liquids held in an absorbent structure and should be compliant, soft feeling and non-irritating.

Nonwovens such as monocomponent spunbond webs often have less than desirable functional performance due to their generally small average pore size, low permeability, and two dimensional nature. Other structures such as bicomponent spunbond webs and through air bonded carded webs can be three dimensional but also tend to have low permeability and small average pore size. The permeability and pore size may be increased through, for example, increased fiber denier and decreased basis weight, but at the extreme limits, softness and other aesthetic features can be compromised. Additionally, under these conditions one often sees a tradeoff in properties, for example, intake rate increases with increase in permeability, but rewet and staining may also increase.

Spunbond webs (SBs) and bonded carded webs (BCWs) used as liners are usually light weight, single layer structures of fine fibers in order to be cost efficient and provide good appearance. However, if these webs have low permeabilities which inhibit fluid flow, they may be treated with surface chemistries (i.e. surfactants) to improve liquid intake. Surfactants usually change the surface tension on the web causing the web to become hydrophilic so that liquid will "wet out" or spread across the surface of the web. This wetting action leaves the liner saturated, prolonging liquid contact with the skin and increasing skin hydration. Some materials are inherently hydrophilic and will also be saturated and promote fluid spreading. It is desirable that personal care articles be designed so as to minimize skin hydration since skin hydration contributes to the occurrence of diaper rash. If the liner has poor liquid intake qualities, remains saturated, or has fluid spreading properties, skin hydration will be increased.

There remains a need, therefore, for a liner with good intake properties, low saturation, and minimal fluid spreading properties, which also has a good appearance and which will reduce skin hydration, and diaper rash accordingly.

## SUMMARY OF THE INVENTION

The object of the invention is achieved by a multi-layer liner for personal care products having at least a top or first web of fibers and a bottom or second web of fibers. There may be additional layers or webs therebetween. The liner has a conductance greater than 100 Darcies/mil and the bottom layer has a capillarity greater than the top layer. The liner should preferably have a basis weight between about 0.2 osy and 1 osy and more particularly between about 0.4 osy and 0.7 osy

## BRIEF DESCRIPTION OF THE DRAWINGS

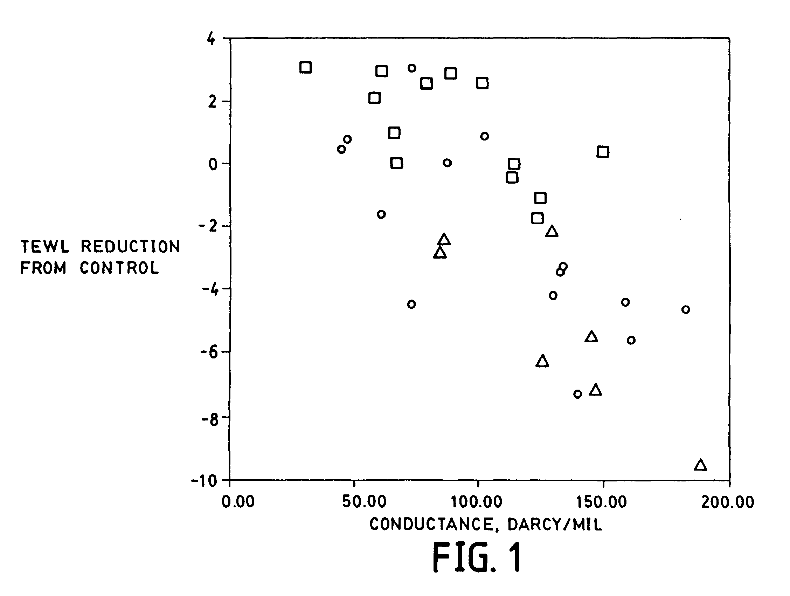

The Figure shows a graph of the general relationship between TEWL reduction versus conductance in webs wherein the circles represent webs which are through air bonded, the triangles represent creped webs, and the squares represent point bonded webs.

## DEFINITIONS

"Disposable" includes being disposed of after a single use and not intended to be washed and reused.

"Front" and "back" are used throughout this description to designate relationships relative to the garment itself, rather than to suggest any position the garment assumes when it is positioned on a wearer.

"Inward" and "outward" refer to positions relative to the center of an absorbent garment, and particularly transversely and/or longitudinally closer to or away from the longitudinal and transverse center of the absorbent garment.

"Liquid" means a non-particulate substance and/or material that flows and can assume the interior shape of a container into which it is poured or placed.

"Liquid communication" means that liquid is able to travel from one layer to another layer, or one location to another within a layer.

"Longitudinal" and "transverse" have their customary meaning. The longitudinal axis lies in the plane of the article when laid flat and fully extended and is generally parallel to a vertical plane that bisects a standing wearer into left and right body halves when the article is worn. The transverse axis lies in the plane of the article generally perpendicular to the longitudinal axis. The article as illustrated is longer in the longitudinal direction than in the transverse direction.

As used herein and in the claims, the term "comprising" is inclusive or open-ended and does not exclude additional unrecited elements, compositional components, or method steps.

As used herein the term "polymer" generally includes but is not limited to, homopolymers, copolymers, such as for example, block, graft, random and alternating copolymers, terpolymers, etc. and blends and modifications thereof. Furthermore, unless otherwise specifically limited, the term "polymer" shall include all possible geometrical configurations of the molecule. These configurations include, but are not limited to isotactic, syndiotactic and random symmetries.

As used herein the term "nonwoven fabric or web" means a web having a structure of individual fibers or threads which are interlaid, but not in an identifiable manner as in a knitted fabric. Nonwoven fabrics or webs have been formed from many processes such as for example, meltblowing processes, spunbonding processes, and bonded carded web processes. The basis weight of nonwoven fabrics is usually expressed in ounces of material per square yard (osy) or grams per square meter (gsm) and the fiber diameters useful are usually expressed in microns. (Note that to convert from osy to gsm, multiply osy by 33.91).

As used herein the term "microfibers" means small diameter fibers having an average diameter not greater than about 75 microns, for example, having an average diameter of from about 0.5 microns to about 50 microns, or more particularly, microfibers may have an average diameter of from about 2 microns to about 40 microns. Another frequently used expression of fiber diameter is denier, which is defined as grams per 9000 meters of a fiber and may be calculated as fiber diameter in microns squared, multiplied by the density in grams/cc, multiplied by 0.00707. A lower denier indicates a finer fiber and a higher denier indicates a thicker or heavier fiber. For example, the diameter of a polypropylene fiber given as 15 microns may be converted to denier by squaring, multiplying the result by 89 g/cc and multiplying by .00707. Thus, a 15 micron polypropylene fiber has a denier of about 1.42 ($15^2$ x 0.89 x .00707 = 1.415). Outside the United States the unit of measurement is more commonly the "tex", which is defined as the grams per kilometer of fiber. Tex may be calculated as denier/9.

As used herein the term "spunbonded fibers" refers to small diameter fibers which are formed by extruding molten thermoplastic material as filaments from a plurality of fine, usually circular capillaries of a spinneret with the diameter of the extruded filaments then being rapidly reduced as by, for example, in US Patent 4,340,563 to Appel et al., and US Patent 3,692,618 to Dorschner et al., US Patent 3,802,817 to Matsuki et al., US Patents 3,338,992 and 3,341,394 to Kinney, US Patent 3,502,763 to Hartman, and US Patent 3,542,615 to Dobo et al. Spunbond fibers are generally not tacky when they are deposited onto a collecting surface. Spunbond fibers are generally continuous and have average diameters (from a sample of at least 10) larger than 7 microns, more particularly, between about 10 and 20 microns. The fibers may also have shapes such as those described in US Patents 5,277,976 to Hogle et al., US Patent 5,466,410 to Hills and 5,069,970 and 5,057,368 to Largman et al., which describe fibers with unconventional shapes.

As used herein the term "meltblown fibers" means fibers formed by extruding a molten thermoplastic material through a plurality of fine, usually circular, die capillaries as molten threads or filaments into converging high velocity, usually hot, gas (e.g. air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers. Such a process is disclosed, for example, in US Patent 3,849,241 to Butin et al. Meltblown fibers are microfibers which may be continuous or discontinuous, are generally smaller than 10 microns in average diameter, and are generally tacky when deposited onto a collecting surface.

"Conjugate fibers" refers to fibers which have been formed from at least two polymers extruded from separate extruders but spun together to form one fiber. Conjugate fibers are also sometimes referred to as multicomponent or bicomponent fibers. The polymers are usually different from each other though conjugate fibers may be monocomponent fibers. The polymers are arranged in substantially constantly positioned distinct zones across the cross-section of the conjugate fibers and extend continuously along the length of the conjugate fibers. The configuration of such a conjugate fiber may be, for example, a sheath/core arrangement wherein one polymer is surrounded by another or may be a side by side arrangement, a pie arrangement or an "islands-in-the-sea" arrangement. Conjugate fibers are taught in US Patent 5,108,820 to Kaneko et al., US Patent 5,336,552 to Strack et al., and US Patent 5,382,400 to Pike et al. For two component fibers, the polymers may be present in ratios of 75/25, 50/50, 25/75 or any other desired ratios. The fibers may also have shapes such as those described in US Patents 5,277,976 to Hogle et al., and 5,069,970 and 5,057,368 to Largman et al., which describe fibers with unconventional shapes.

"Biconstituent fibers" refers to fibers which have been formed from at least two polymers extruded from the same extruder as a blend. The term "blend" is defined below. Biconstituent fibers do not have the various polymer components arranged in relatively constantly positioned distinct zones across the cross-sectional area of the fiber and the various polymers are usually not continuous along the entire length of the fiber, instead usually forming fibrils or protofibrils which start and end at random. Biconstituent fibers are sometimes also referred to as multiconstituent fibers. Fibers of this general type are discussed in, for example, US Patent 5,108,827 to Gessner. Bicomponent and biconstituent fibers are also discussed in the textbook Polymer Blends and Composites by John A. Manson and Leslie H. Sperling, copyright 1976 by Plenum Press, a division of Plenum Publishing Corporation of New York, IBSN 0-306-30831-2, at pages 273 through 277.

"Bonded carded web" refers webs are made from staple fibers which are sent through a combing or carding unit, which breaks apart and aligns the staple fibers in the machine direction to form a generally machine direction-oriented fibrous nonwoven web. Such fibers are usually purchased in bales which are placed in a picker which separates the fibers prior to the carding unit. Once the web is formed, it then is bonded by one or more of several known bonding methods. One such bonding method is powder bonding, wherein a powdered adhesive is distributed through the web and then activated, usually by heating the web and adhesive with hot air. Another suitable bonding method is pattern bonding, wherein heated calender rolls or ultrasonic bonding equipment are used to bond the fibers together, usually in a localized bond pattern, though the web can be bonded across its entire surface if so desired. Another suitable and well-known bonding method, particularly when using bicomponent staple fibers, is through-air bonding.

"Airlaying" is a well known process by which a fibrous nonwoven layer can be formed. In the airlaying process, bundles of small fibers having typical lengths ranging from about 6 to about 19 millimeters (mm) are separated and entrained in an air supply and then deposited onto a forming screen, usually with the assistance of a vacuum supply. The randomly deposited fibers then are bonded to one another using, for example, hot air or a spray adhesive.

As used herein, through-air bonding or "TAB" means a process of bonding a nonwoven fiber web in which air which is sufficiently hot to melt or partially melt the polymer of which the fibers are made. The air velocity is between 30.5-152 metres (100 and 500 feet) per minute and the dwell time may be as long as 6 seconds. The melting and resolidification of the polymer provides the bonding. Through air bonding has relatively restricted variability and since through-air bonding requires the melting of at least one component to accomplish bonding, it is restricted to webs with two components like conjugate fibers or those which include an adhesive. In the through-air bonder, air having a temperature above the melting temperature of one component and below the melting temperature of another component is directed from a surrounding hood, through the web, and into a perforated roller supporting or withdrawal means. Alternatively, the through-air bonder may be a flat arrangement wherein the air is directed vertically downward onto the web. The operating conditions of the two configurations are similar, the primary difference being the geometry of the web during bonding. The hot air melts the lower melting polymer component and thereby forms bonds between the filaments to integrate the web.

As used herein, the term "stitchbonded" means, for example, the stitching of a material in accordance with US Patent 4,891,957 to Strack et al. or US Patent 4,631,933 to Carey, Jr.

As used herein, "ultrasonic bonding" means a process performed, for example, by passing the fabric between a sonic horn and anvil roll as illustrated in US Patent 4,374,888 to Bornslaeger.

As used herein "thermal point bonding" involves passing a fabric or web of fibers to be bonded between a heated calender roll and an anvil roll. The calender roll is usually, though not always, patterned in some way so that the entire fabric is not bonded across its entire surface, and the anvil roll is usually flat. As a result, various patterns for calender rolls have been developed for functional as well as aesthetic reasons. One example of a pattern has points and is the Hansen Pennings or "H&P" pattern with about a 30% bond area with about 31 bonds/square centimetre (200 bonds/square inch) as taught in U.S. Patent 3,855,046 to Hansen and Pennings. The H&P pattern has square point or pin bonding areas wherein each pin has a side dimension of 0.038 inches (0.965 mm), a spacing of 0.070 inches (1.778 mm) between pins, and a depth of bonding of 0.023 inches (0.584 mm). The resulting pattern has a bonded area of about 29.5%. Another typical point bonding pattern is the expanded Hansen Pennings or "EHP" bond pattern which produces a 15% bond area with a square pin having a side dimension of 0.037 inches (0.94 mm), a pin spacing of 0.097 inches (2.464 mm) and a depth of 0.039 inches (0.991 mm). Another typical point bonding pattern designated "714" has square pin bonding areas wherein each pin has a side dimension of 0.023 inches, a spacing of 0.062 inches (1.575 mm) between pins, and a depth of bonding of 0.033 inches (0.838 mm). The resulting pattern has a bonded area of about 15%. Yet another common pattern is the C-Star pattern which has a bond area of about 16.9%. The C-Star pattern has a cross-directional bar or "corduroy" design interrupted by shooting stars. Other common patterns include a diamond pattern with repeating and slightly offset diamonds with about a 16% bond area and a wire weave pattern looking as the name suggests, e.g. like a window screen, with about a 19% bond area. Typically, the percent bonding area varies from around 10% to around 30% of the area of the fabric laminate web. As in well known in the art, the spot bonding holds the laminate layers together as well as imparts integrity to each individual layer by bonding filaments and/or fibers within each layer.

As used herein "pattern unbonded" or interchangeably "point unbonded" or "PUB", means a fabric pattern having continuous bonded areas defining a plurality of discrete unbonded areas. The fibers or filaments within the discrete unbonded areas are dimensionally stabilized by the continuous bonded areas that encircle or surround each unbonded area, such that no support or backing layer of film or adhesive is required. The unbonded areas are specifically designed to afford spaces between fibers or filaments within the unbonded areas. A suitable process for forming the pattern-unbonded nonwoven material of this invention includes providing a nonwoven fabric or web, providing oppositely positioned first and second calender rolls and defining a nip therebetween, with at least one of said rolls being heated and having a bonding pattern on its outermost surface comprising a continuous pattern of land areas defining a plurality of discrete openings, apertures or holes, and passing the nonwoven fabric or web within the nip formed by said rolls. Each of the openings in said roll or rolls defined by the continuous land areas forms a discrete unbonded area in at least one surface of the nonwoven fabric or web in which the fibers or filaments of the web are substantially or completely unbonded. Stated alternatively, the continuous pattern of land areas in said roll or rolls forms a continuous pattern of bonded areas that define a plurality of discrete unbonded areas on at least one surface of said nonwoven fabric or web. Alternative embodiments of the aforesaid process includes pre-bonding the nonwoven fabric or web before passing the fabric or web within the nip formed by the calender rolls, or providing multiple nonwoven webs to form a pattern-unbonded laminate.

"Personal care product" means diapers, training pants, swim wear, absorbent underpants, adult incontinence products, bandages and feminine hygiene products.

"Feminine hygiene products" means sanitary napkins or pads, tampons and panty-liners.

"Target area" refers to the area or position on a personal care product where an insult is normally delivered by a wearer.

## TEST METHODS AND MATERIALS

Basis Weight: A sample of 3" (7.6 cm) diameter circle is cut and weighed using a balance. Weight is recorded in grams. The weight is divided by the sample area. Five samples are measured and averaged.

Density The density of the materials is calculated by dividing the weight per unit area of a sample in grams per square meter (gsm) by the material caliper. A total of three samples would be evaluated and averaged for the density values.

Permeability: Permeability is obtained from a measurement of the resistance by the material to the flow of liquid. A liquid of known viscosity is forced through the material of a given thickness at a constant flow rate and the resistance to flow, measured as a pressure drop is monitored. Darcy's Law is used to determine permeability as follows:

$$\text{Permeability} = [\text{flow rate} \times \text{thickness} \times \text{viscosity} / \text{pressure drop}] \qquad \text{[Equation 1]}$$

where the units are:

| | | |
|---|---|---|
| permeability: | $cm^2$ or Darcy | 1 Darcy = 9.87 x $10^{-9}$ $cm^2$ |
| flow rate: | cm/sec | |
| viscosity: | Pascal-sec | |
| pressure drop: | Pascals | |

The apparatus consists of an arrangement wherein a piston within a cylinder pushes liquid through the sample to be measured. The sample is clamped between two aluminum cylinders with the cylinders oriented vertically. Both cylinders have an outside diameter of 8.9 cm (3.5"), an inside diameter of 6.35 cm (2.5") and a length of about 15.2 cm (6"). The 7.62 cm (3") diameter web sample is held in place by its outer edges and hence is completely contained within the apparatus. The bottom cylinder has a piston that is capable of moving vertically within the cylinder at a constant velocity and is connected to a pressure transducer that capable of monitoring the pressure of encountered by a column of liquid supported by the piston. The transducer is positioned to travel with the piston such that there is no additional pressure measured until the liquid column contacts the sample and is pushed through it. At this point, the additional pressure measured is due to the resistance of the material to liquid flow through it. The piston is moved by a slide assembly that is driven by a stepper motor. The test starts by moving the piston at a constant velocity until the liquid is pushed through the sample. The piston is then halted and the baseline pressure is noted. This corrects for sample buoyancy effects. The movement is then resumed for a time adequate to measure the new pressure. The difference between the two pressures is the pressure due to the resistance of the material to liquid flow and is the pressure drop used in Equation (1). The velocity of the piston is the flow rate. Any liquid whose viscosity is known can be used, although a liquid that wets the material is preferred since this ensures that saturated flow is achieved. The measurements were carried out using a piston velocity of 20 cm/min, mineral oil (Peneteck Technical Mineral Oil manufactured by Penreco of Los Angeles, CA) of a viscosity of 6 centipoise.

Alternatively, permeability can be calculated from the following equation:

$$\text{Permeability} = 0.051 * R * (1 - \text{Porosity}) * (\text{Porosity}/(1 - \text{Porosity}))^{2.75} \qquad \text{[Equation 2]}$$

where R = fiber radius
and

$$\text{Porosity} = 1 - (\text{web density}/\text{fiber density}) \qquad \text{[Equation 3]}$$

Reference for Equation 2 can be found in the article "Quantification of Unidirectional Fiber Bed Permeability by J. Westhuizen and J.P. Du Plessis in the Journal of Composite Materials, 28(7), 1994. Note that the equations show that permeability can be determined if fiber radius, web density and fiber density are known.

Material caliper (thickness): The caliper of a material is a measure of thickness and is measured at 345 Pa (0.05 psi) psi) with a Starret-type bulk tester with an acrylic platen, in units of millimeters. In practice, 10 repetitions of any measurement should be made and averaged.

TransEpidermal Water Loss (TEWL):

Skin hydration values are determined by measuring TransEpidermal Water Loss (TEWL) and can be determined by employing the following test procedure.

The test is conducted on adults on the forearm. Any medications should be reviewed to ensure they have no effect on test results and the subject's forearms should be free of any skin conditions such as rashes or abrasions. Subjects should relax in the test environment, which should be at about 72°F (22 °C) with a humidity of about 40 percent, for about 15 minutes prior to testing and movement should be kept to a minimum during testing. Subjects should wear short sleeve shirts, not bathe or shower for about 2 hours before testing, and should not apply any perfumes, lotions, powders, etc., to the forearm.

The measurements are taken with an evaporimeter, such as an Evaporimeter EP1 instrument distributed by Servomed AB, Stockholm, Sweden.

A baseline reading should be taken on the subject's forearm and should be less than 10 $g/m^2/hr$. Each test measurement is taken over a period of two minutes with TEWL values taken once per second (a total of 120 TEWL values). The digital output from the Evaporimeter EP1 instrument gives the rate of evaporative water loss (TEWL) in $g/m^2/hr$.

For testing, the end of a dispensing tube is placed on the mid-forearm. An armband is placed on the subject's forearm directly over the end of the tube. The eye of the tube should be facing the target loading zone.

Hold the product in place with tape without allowing it to come in contact with the subject's skin. For the testing herein, the product was a HUGGIES ULTRATRIM Step 3 diaper having 8.9 gms of superabsorbent placed in a zone about 2.5 inches (63.6 mm) wide and the standard liner was replaced with the web to be tested.

Place a stretchable net such as that available from Zens Industrial Knit Products of Milwaukee, WI, over the product to help to hold it in place.

Three equal loadings of 60 ml of physiologic saline available from VWR Scientific Products (800-932-5000) at about 95 °F (35 °C) are delivered to the product at an interval of 45 seconds by a pump such as a MASTERFLEX Digi-Static batch/dispense pump. After 60 minutes, the product is removed from the subject's forearm and Evaporimeter readings taken immediately on the skin where the product had been.

TransEpidermal Water Loss values are reported as the difference between the one hour and baseline values in $g/m^2/hr$.

Conductance: This is calculated as permeability per unit thickness and gives a measure of the openness of a particular structure and so an indication of the relative ease at which a material will transmit liquid. The Figure is a graph of TEWL reduction versus conductance with conductance on the X-axis with each division representing 0.0195 $cm^2$/m (50 Darcies/mil) and TEWL reduction in units of percentage change on the Y-axis.

Capillarity: Capillarity is defined as the tendency of liquids to be absorbed by and held within a porous structure. This tendency, expressed as capillary pressure, is quantified by the following equation:

$$\text{Capillary pressure} = 2 \times \text{Gamma} \times \cos\theta / r_{effective} \qquad \text{[Equation 4]}$$

Gamma = surface tension of liquid
$\theta$ = contact angle of liquid with solid, a measure of wettability
Porosity = 1 - (web density/fiber density)
For fibrous structures, the effective pore radius, $r_{effective}$ can be calculated from the following:

$$r_{effective} = \text{Porosity} \times \text{fiber diameter} / 4 \times (1- \text{porosity}) \qquad \text{[Equation 5]}$$

## DETAILED DESCRIPTION OF THE INVENTION

This invention relates to a topsheet or liner when used in an absorbent article to provide enhanced skin dryness.

A liner should have intake properties such that the incoming liquid stream is transported through the material and hence, minimal pooling of the liquid at the top surface occurs. Additionally, the top layer of the liner should have minimal saturation so that the skin hydration is not increased. This can be accomplished by providing a structure that has a conductance to liquid of greater than or equal to 0.039 $cm^2$/m (100 darcys/mil) thickness and that possesses a capillary gradient such that the capillarity of the top surface layer is lower than the bottom layer.

The inventive dry liner is a multiple layer web where the top surface of the web is porous and ideally, hydrophobic and the bottom surface of the web is porous and hydrophilic. The capillarity of the top layer needs to be lower than the other layers. This will always be true for a hydrophobic top layer regardless of its pore size, otherwise the capillary equation is used to establish the correct capillary gradient. The bottom hydrophilic layer helps pull the liquid from the hydrophobic layer to the next layer of the absorbent article. There must be intimate contact between the layers as well as between the liner and the next layer in the absorbent structure. This assures that proper draining of the top layer occurs for minimal saturation. By "intimate contact" what is meant is that there is sufficient contact for liquid to transfer easily between the layers.

According to the bridging pressure equation given below, if the hydrophobic layer is thin (t is small), and porous (R is large), then liquid will pass through the layer. The bridging pressure must be smaller than the hydrostatic head exerted by the liquid in order for the liquid to pass through. A capillarity gradient alone is not sufficient for reducing skin hydration. High conductance and minimal fluid spreading must also be considered.

Bridging pressure equation:

$$P_{bridge} = 4 \times \gamma \times t / (r_{effective}^2 + t^2)$$

where;

$y$ = surface tension of fluid
$r_{effective}$ = effective pore size
t = thickness of hydrophobic layer

The layers may be nonwoven webs made according to various processes known in the art including spunbonding, carding and airlaying. The webs may be bonded according to known processes as well, including through air bonding, stitchbonding, ultrasonic bonding, point bonding, and pattern (or point) unbonding.

Polymers useful in the manufacture of the webs of the invention include thermoplastic polymers like polyolefins, polyesters and polyamides. Elastic polymers may also be used and include block copolymers such as polyurethanes, copolyether esters, polyamide polyether block copolymers, ethylene vinyl acetates (EVA), block copolymers having the general formula A-B-A' or A-B like copoly(styrene/ethylene-butylene), styrene-poly(ethylene-propylene)-styrene, styrene-poly(ethylene-butylene)-styrene, (polystyrene/poly(ethylene-butylene)/polystyrene, poly(styrene/ethylene-butylene/styrene) and the like. Polyolefins using single site catalysts, sometimes referred to as metallocene catalysts, may also be used.

Many polyolefins are available for fiber production, for example polyethylenes such as Dow Chemical's ASPUN® 6811A linear low density polyethylene, 2553 LLDPE and 25355 and 12350 high density polyethylene are such suitable polymers. The polyethylenes have melt flow rates, respectively, of about 26, 40, 25 and 12. Fiber forming polypropylenes include Exxon Chemical Company's Escorene® PD 3445 polypropylene and Montell Chemical Co.'s PF-304. Many other polyolefins are commercially available. The fibers used to produce the webs useful in this invention may be monocomponent, conjugate (bicomponent) or biconstituent fibers. If conjugate, they may have side-by-side, sheath/core or islands-in-the-stream configurations. The fibers may be crimped or crimpable according to, for example, US Patent 5,382,400 to Pike.The basis weight of the webs may be between about 0.2 osy (6.8 gsm) and 1 osy (33.9 gsm) or more particularly between about 0.4 osy (13.6 gsm) and 0.7 osy (23.7 gsm). The basis weight of the hydrophobic top layer should be such that the bridging pressure equation is satisfied. That is, the appropriate bridging pressure to cause liquid to penetrate the hydrophobic layer spontaneously because of its hydrostatic head is used to determine its effective pore size and thickness. Equation 5 can be used to determine effective pore size given web density and fiber size and thickness can be determined by dividing basis weight by web density. For most applications, a hydrostatic head of less than 1 cm is used. Additionally, there must be intimate contact between the layers which may be accomplished by the web forming process, through the bonding of the layers as discussed above, and through the application of pressure.

It has been found that the inventive liner must have a conductance above 0.039 $cm^2/m$ (100 Darcies per mil) of thickness. Experimental data show that the TransEpidermal Water Loss (TEWL) significantly decreases when the conductance is above the critical level of 0.039 $cm^2/m$ (100 Darcies/mil).

The capillarity difference between the two layers also increases intake and minimizes saturation.

The following Examples illustrate the advantages of the invention. Note that in the Examples, reductions in TEWL numbers are relative to a control. The control was a 0.5 osy, 2.2 denier polypropylene spunbond web with wireweave point bonding pattern. The control also had a treatment of 0.3 weight percent AHCOVEL surfactant. The control had a thickness of 0.009 inches (0.2 mm), a density of 0.071 g/cc, a permeability of about 640 Darcies and a conductance of about 0.0273 $cm^2/m$ (70 Darcies/mil). The results of testing of the control and Example webs may be found in Table 1.

## Example 1

A spunbond web was produced having a side-by-side conjugate fiber configuration. One half of the fiber was made from Exxon's 3155 polypropylene and the other from Dow's ASPUN 6811 LLDPE. The basis weight of the web was 0.7 osy with 0.3 osy in the top layer and 0.4 osy in the bottom layer for a ratio of 43: 57. The top layer fibers had a denier of about 2.5 and the bottom layer about 6. The bottom layer also included about 1.25 weight percent of PPG Corporation's MASIL SF-19 internal surfactant.

The layers were produced together with the top layer being made directly onto the bottom layer and then the layers were through air bonded.

The web so produced was tested according to the bulk, density and TEWL methods. The bulk of the web was about 0.033 inches (0.84 mm). The density was about 0.028 g/cc, and the change in TEWL was about = -21.8%. The permeability was about 5570 Darcies and the conductance about 0.064 $cm^2/m$ (164 Darcies/mil).

## Example 2

A thermally bonded carded web comprised of blended staple fibers was produced. Fibers within this example were produced by KoSa and Chisso Corporation and included sheath/core bicomponent fibers as well as polyester fibers. The overall basis weight was 0.6 osy with 0.3 osy in the top layer and 0.3 in the bottom for a 50:50 layer ratio. The top layer was comprised of 100% bicomponent Type 256 staple fibers, merge 35103A, from KoSa, with a hydrophobic topcoat finish. The bottom layer contained 60% Chisso Type 233 bicomponent fibers from Chisso with HR6 hydrophilic finish and 40% Type 295 polyester fiber merge number 635020, with a hydrophilic finish, from KoSa. This layered web was produced in line on using a multiple card set up were each layer was formed separately then brought together and thermally bonded.

The web so produced was tested according to the bulk, density and TEWL methods. The bulk of the web was about 0.034 inches (0.86 mm). The density was about 0.025 g/cc, and the change in TEWL was about = -23.3%. The permeability was about 4750 Darcies and the conductance about 0.055 $cm^2/m$ (140 Darcies/mil).

### Example 3

A bonded carded web was produced. The basis weight of the web was 0.7 osy with 0.3 osy in the top layer and 0.4 osy in the bottom layer for a ratio of 43:57. The top layer fibers had a denier of about 3 and was made from KoSa T-256 fibers. The top layer also was treated with 0.3 weight percent AHCOVEL surfactant available from ICI Chemicals. The bottom layer was the same mixture fibers as in Example 2.

The layers were produced according to the carding process and then through air bonded.

The web so produced was tested according to the bulk, density and permeability methods. The bulk of the web was about 0.041 inches (1.04 mm). The density was about 0.024 g/cc. The permeability was about 6845 Darcies and the conductance about 0.066 cm$^2$/m (170 Darcies/mil)

Such a web is believed useful for feminine hygiene applications and so was not tested according to the TEWL method.

### Example 4

A thermally bonded carded web comprised of blended staple fibers was produced. Fibers within this example were produced by KoSa and Chisso Corporation and included sheath/core bicomponent fibers as well as polyester fibers. The overall basis weight was 0.6 osy with 0.3 osy in the top layer and 0.3 in the bottom for a 50:50 layer ratio. The top layer was comprised of 50% bicomponent Type 256 merge 35103A staple fibers with a hydrophobic topcoat finish and 50% bicomponent Type 256 merge 34687A staple fibers with a hydrophilic topcoat finish, both from KoSa. The bottom layer contained 60% Chisso Type 233 bicomponent fibers from Chisso with HR6 hydrophilic finish and 40% Type 295 polyester fiber merge number 635020, with a hydrophilic finish, from KoSa. This layered web was produced in line on using a multiple card set up were each layer was formed separately then brought together and thermally bonded.

The web so produced was tested according to the bulk, density and TEWL methods. The bulk of the web was about 0.034 inches (0.86 mm). The density was about 0.025 g/cc, and the change in TEWL was about = -12%. The permeability was about 4750 Darcies and the conductance about 0.055 cm$^2$/m (140 Darcies/mil).

This Example 4 shows that the top layer need not be made entirely from hydrophobic fibers but may be made from a blend of hydrophobic and hydrophilic fibers and still function well.

**Table 1**

| Specimen | TEWL | TEWL reduction | % TEWL reduction |
|----------|------|----------------|------------------|
| Control | 33.2 | NA | NA |
| Example 1 | 25.9 | -7.2 | -21.8 |
| Example 2 | 25.5 | -7.7 | -23.3 |
| Example 4 | 29.2 | -4 | -12 |

Although only a few exemplary embodiments of this invention have been described in detail above, those skilled in the art will readily appreciate that many modifications are possible in the exemplary embodiments without materially departing from the novel teachings and advantages of this invention. Accordingly, all such modifications are intended to be included within the scope of this invention as defined in the following

**Claims**

1. A multi-layer liner for personal care products comprising at least a first, top web of fibers and a second, bottom web of fibers, wherein said liner has a conductance greater than 0.039 cm$^2$/m (100 Darcies/mil), and said second web has a capillarity greater than said first web.

2. The liner of claim 1 wherein said liner has a basis weight between about 6.8 g/m$^2$ (0.2 osy) and 34 g/m$^2$ (1 osy).

3. The liner of claim 1 wherein said liner has a basis weight between about 14 g/m$^2$ (0.4 osy) and 24 g/m$^2$ (0.7 osy).

4. The liner of claim 1 wherein said top web is hydrophobic and said bottom web is hydrophilic.

5. The liner of claim 1 wherein said top web is comprised of hydrophobic and hydrophilic fibers and said bottom web is hydrophilic.

6. The liner of claim 1 wherein said liner has a total thickness between about 0.2 mm and 4.0 mm.

7. The liner of claim 1 wherein said fibers of said top web are made from polyolefin.

8. The liner of claim 7 wherein said polyolefin is selected from the group consisting of polyethylene, polypropylene, polybutylene and copolymers and blends thereof.

9. A diaper comprising the liner of claim 1.

10. The diaper of claim 9 wherein said liner is oriented such that a side away from a wearer is hydrophobic.

11. The diaper of claim 9 wherein said liner is oriented such that a side toward a wearer is hydrophobic.

12. The diaper of claim 9 which further comprises a breathable outercover.

13. A training pant comprising the liner of claim 1.

14. An incontinence product comprising the liner of claim 1.

15. A bandage comprising the liner of claim 1.

16. A sanitary napkin comprising the liner of claim 1.

17. A nursing pad comprising the liner of claim 1.


**Patentansprüche**

1. Mehrschichtdecklage für Körperpflegeprodukte, umfassend mindestens eine erste Oberbahn von Fasern und eine zweite Unterbahn von Fasern, wobei die Decklage eine Konduktanz größer als 0,039 $cm^2$/m (100 Darcies/Mil) aufweist, und die zweite Bahn eine Kapillarität größer als die der ersten Bahn aufweist.

2. Decklage nach Anspruch 1, wobei die Decklage ein Flächengewicht zwischen etwa 6,8 $g/m^2$ (0,2 osy) und 34 $g/m^2$ (1 osy) aufweist.

3. Decklage nach Anspruch 1, wobei die Decklage ein Flächengewicht zwischen etwa 14 $g/m^2$ (0,4 osy) und 24 $g/m^2$ (0,7 osy) aufweist.

4. Decklage nach Anspruch 1, wobei die Oberbahn hydrophob ist und die Unterbahn hydrophil ist.

5. Decklage nach Anspruch 1, wobei die Oberbahn hydrophobe und hydrophile Fasern umfasst und die Unterbahn hydrophil ist.

6. Decklage nach Anspruch 1, wobei die Decklage eine Gesamtdicke zwischen etwa 0,2 mm und 4,0 mm aufweist.

7. Decklage nach Anspruch 1, wobei die Fasern der Oberbahn aus Polyolefin hergestellt sind.

8. Decklage nach Anspruch 7, wobei das Polyolefin aus der Gruppe, bestehend aus Polyethylen, Polypropylen, Polybutylen und Copolymeren und Blends davon, ausgewählt ist.

9. Windel, umfassend die Decklage von Anspruch 1.

10. Windel nach Anspruch 9, wobei die Decklage derart orientiert ist, dass eine Seite entfernt von einem Träger hydrophob ist.

11. Windel nach Anspruch 9, wobei die Decklage derart orientiert ist, dass eine Seite zu einem Träger hydrophob ist.

**12.** Windel nach Anspruch 9, die weiterhin eine luftdurchlässige Außenschicht umfasst.

**13.** Windelhose, umfassend die Decklage von Anspruch 1.

**14.** Inkontinenzprodukt, umfassend die Decklage von Anspruch 1.

**15.** Verband, umfassend die Decklage von Anspruch 1.

**16.** Damenbinde, umfassend die Decklage von Anspruch 1.

**17.** Stilleinlage, umfassend die Decklage von Anspruch 1.

**Revendications**

**1.** Doublure multicouche pour produits d'hygiène personnelle comprenant au moins un premier voile supérieur de fibres et un second voile inférieur de fibres, ladite doublure ayant une conductance supérieure à 0,039 cm$^2$/m (100 Darcy/millième de pouce), et ledit second voile ayant une capillarité supérieure à celle dudit premier voile.

**2.** Doublure selon la revendication 1, où ladite doublure a une masse surfacique comprise entre environ 6,8 g/m$^2$ (0,2 osy) et 34 g/m$^2$ (1 osy).

**3.** Doublure selon la revendication 1, où ladite doublure a une masse surfacique comprise entre environ 14 g/m$^2$ (0,4 osy) et 24 g/m$^2$ (0,7 osy).

**4.** Doublure selon la revendication 1, dans laquelle ledit voile supérieur est hydrophobe et ledit voile inférieur est hydrophile.

**5.** Doublure selon la revendication 1, dans laquelle ledit voile supérieur est composé de fibres hydrophobes et hydrophiles et ledit voile inférieur est hydrophile.

**6.** Doublure selon la revendication 1, où ladite doublure a une épaisseur totale comprise entre environ 0,2 mm et 4,0 mm.

**7.** Doublure selon la revendication 1, dans laquelle lesdites fibres dudit voile supérieur sont faites à partir d'une polyoléfine.

**8.** Doublure selon la revendication 7, dans laquelle ladite polyoléfine est sélectionnée dans le groupe consistant en le polyéthylène, le polypropylène, le polybutylène, ainsi que les copolymères et mélanges de ceux-ci.

**9.** Couche-culotte comprenant la doublure selon la revendication 1.

**10.** Couche-culotte selon la revendication 9, dans laquelle ladite doublure est orientée de telle sorte qu'une face, à l'écart d'un porteur, est hydrophobe.

**11.** Couche-culotte selon la revendication 9, dans laquelle ladite doublure est orientée de telle sorte qu'une face, vers un porteur, est hydrophobe.

**12.** Couche-culotte selon la revendication 9, qui comprend en outre une feuille extérieure de couverture respirante.

**13.** Culotte d'apprentissage de la propreté comprenant la doublure selon la revendication 1.

**14.** Produit pour incontinence comprenant la doublure selon la revendication 1.

**15.** Pansement comprenant la doublure selon la revendication 1.

**16.** Serviette hygiénique comprenant la doublure selon la revendication 1.

**17.** Coussinet d'allaitement comprenant la doublure selon la revendication 1.

FIG. 1

**EP 1 154 744 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4340563 A
- US 3692618 A, Dorschner
- US 3802817 A, Matsuki
- US 3338992 A
- US 3341394 A, Kinney
- US 3502763 A, Hartman
- US 3542615 A, Dobo
- US 5277976 A, Hogle
- US 5466410 A, Hills
- US 5069970 A
- US 5057368 A, Largman
- US 3849241 A
- US 5108820 A, Kaneko
- US 5336552 A, Strack
- US 5382400 A, Pike
- US 5108827 A, Gessner
- US 4891957 A, Strack
- US 4631933 A, Carey, Jr
- US 4374888 A
- US 3855046 A, Hansen and Pennings

**Non-patent literature cited in the description**

- **JOHN A. MANSON ; LESLIE H. SPERLING.** Polymer Blends and Composites. Plenum Press, 1976, 273-277
- **J. WESTHUIZEN ; J.P. DU PLESSIS.** Quantification of Unidirectional Fiber Bed Permeability. *Journal of Composite Materials,* 1994, vol. 28 (7